**Europäisches Patentamt**

**European Patent Office**

**Office européen des brevets**

(19)

(11) Publication number : **0 359 259 B1**

(12) **EUROPEAN PATENT SPECIFICATION**

(45) Date of publication of patent specification :
19.08.92 Bulletin 92/34

(51) Int. Cl.⁵ : **A61K 31/66**

(21) Application number : **89117031.8**

(22) Date of filing : **14.09.89**

(54) **The use of inositoltrisphosphate for the preparing of a medicament against transplantation disorders.**

(30) Priority : **15.09.88 SE 8803248**

(43) Date of publication of application :
**21.03.90 Bulletin 90/12**

(45) Publication of the grant of the patent :
**19.08.92 Bulletin 92/34**

(84) Designated Contracting States :
**AT BE CH DE ES FR GB GR IT LI LU NL SE**

(56) References cited :
**EP-A- 0 179 439**
**EP-A- 0 252 227**

(73) Proprietor : **PERSTORP AB**
**S-28480 Perstorp (SE)**

(72) Inventor : **Sirén, Matti**
**Via Poporino 9**
**CH-6926 Montagnola Lugano (CH)**

(74) Representative : **Hansen, Bernd, Dr.rer.nat. et al**
**Hoffmann, Eitle & Partner Patentanwälte**
**Arabellastrasse 4 Postfach 81 04 20**
**W-8000 München 81 (DE)**

## Description

The present invention relates to the use of at least one isomer of inositoltrisphosphate ($IP_3$) for the preparing of a medicament for preventing, alleviating or combatting disorders related to transplantations in mammals including man.

Transplantation or grafting of different organs or tissues to other areas on the body or to other individuals are of increasing incidence and importance. Improved surgical techniques and a better understanding of immunology problems are factors which contribute to the increasing importance.

Transplantation of different organs and tissues such as hearts, kidneys, livers, bone marrow and skin are today quite common with kidney grafting being the most frequent one.

Although transplantations have increased lately there is still a large potential for many more successful transplantations if some main problems can be solved.

When an organ, which is to be transplanted, is taken out from the donor the blood flow is decreased and then stopped. During this period of ischemia many conditions are changed, which are detrimental to the organ and the function of the organ when introduced into the acceptor. Among other things the oxygen pressure is changed, which affects the appearance of oxygen-containing species. These species causes damages to constituents in the cell membrane, which deteriorates the function of the different cells in the tissue.

After the organ is taken out from the donor there is always a period of time before the organ is transferred to the acceptor. During this time period the organ is preserved in either 37°C or in cold. Damage to the organ during this period decreases the function and the chance of survival of the organ. This means that in most cases the time between taking out the organ from the donor to grafting it into the acceptor must be very short. These conditions are of course very limiting to increasing the number of transplantations and for the performed transplantations to be successful.

When introducing a new organ into the acceptor there is a phase when the blood starts to flow through the new organ. This reperfusion period is also very severe to the vitality and function of the organ. Also in this case there is an increased occurrence of reactive compounds, which negatively affects the organ.

Another critical component when grafting an organ into an acceptor is the appearance of immunological problems. Many transplants are more or less rejected when introduced into a new body, which results in impairment of the function or in worst case rejection of the organ.

No treatment for reducing the effects during the periods of ischemia or reperfusion exists today.

For preservation of the organs before being transplanted into the acceptor the common method used today is to reduce the temperature to approximately 0°C.

Some compounds, such as vitamin E, have been used in order to be able to prolong the storage time. Still there is a need for a treatment which preserves the organs for a longer time without loss of function.

In order to reduce the immunological characteristics, steroids in combination with immunosuppressive agents, like cyclosporin, has been used. However, this treatment has severe side effects as e.g. cyclosporin is nephrotoxic.

In order to improve the function of transplanted organs and diminish the tendency of rejection there is a need for improved treatments.

According to the present invention it has surprisingly become possible to meet the above need by using at least one isomer of inositoltrisphosphate ($IP_3$) for the preparing of a medicament for preventing, alleviating or combatting disorders related to transplantation in mammals including man.

From the European Patent No. 179439 a pharmaceutical composition comprising as a pharmaceutically active ingredient at least one isomer of inositoltrisphosphate is known. In said patent the effect of this pharmaceutical composition is shown for different areas, such as cardiovascular diseases.

The present invention relates to the use of $IP_3$ for preventing, alleviating or combatting disorders related to transplantation, such as reducing or combatting the ischemic and reperfusion damages; increasing the preservation time; improving the function of the transplanted tissue or organ and reducing immunological reactions leading to rejection.

The invention also relates to reducing or combatting damages of ischemia and reperfusion where these conditions appear in diseases and condition not primarily related to transplantations i.e. other types of graft operations, by-pass surgery, different types of infarctions and stroke.

The production of $IP_3$ and the isolation of the different isomers thereof are disclosed in the US patent No 4.777.134. The $IP_3$ isomers can also be produced by synthetic methods, chemically or enzymatically, starting with e.g. inositol and a phosphorus source. Furthermore, microbiological production methods including hybrid DNA-techniques of $IP_3$ are also suitable.

The structure of $IP_3$ and the different isomers thereof are disclosed in the US patent No 4.735.936 and the US patent No 4.797.390.

It is suitable that the medicament used according to the invention exists in unit dosage form. Tablets, granules or capsules are suitable administration forms for such unit dosage. Furthermore, tablets and granules can easily be surface treated such as to provide an enteric coating to prevent an uncontrolled hydrolysis in the stomach and to bring about a desired absorption in the intestine. Other suitable administration forms are slow release and transdermal administration. A usual pharmaceutically acceptable additive, excipient and/or carrier can be included in the medicament. The tablets or granules can also contain a disintegrant which causes the tablets or the granules, respectively, to disintegrate easily in the intestine. In certain cases, especially in acute situations, it is preferable to use the unit dosage in the form of a solution for intravenous administration.

The medicament can also consist as such of $IP_3$ solely without any additive, excipient or carrier.

If desired, the medicament can be free of other inositol phosphates, $IP_1$, $IP_2$, $IP_4$, $IP_5$ and $IP_6$. Accordingly, the mixture of $IP_3$ isomers can have a purity of 90-100%, such as 93-100% or preferably 95-100%.

Alternatively, the medicament can consist of or comprise one or more specific $IP_3$ isomers, each present in substantially pure form. Thus, the different isomers can be isolated from each other in substantially pure form, which means that they have a purity of 80-100%, such as 82-100% or 85-100%, preferably 90-100%. Since the isomers can be produced in pure form they can be mixed in any proportion, of course.

The medicament can consist of $IP_3$, wherein said $IP_3$ is provided by at least one of $IP_6$, $IP_5$ or $IP_4$ and a degradative substance such as an enzyme suitable to form $IP_3$.

It is in most cases suitable that the $IP_3$-isomer or isomers used for the preparing of the medicament according to the invention is present in salt form in order not to affect the mineral balance negatively. The salt should preferably consist of a sodium, potassium, calcium, zinc or magnesium salt or a mixture of two or more of these salts. Calcium and zinc salts or mixtures of these are especially preferred. The isomer of $IP_3$ can also partly be present as a salt of one or more physiologically acceptable compounds in the lanthanide series.

For the above mentioned reasons it is also an advantage if the medicament contains a surplus or an extra addition of at least one pharmaceutically acceptable salt of calcium, zinc or magnesium with a mineral acid or organic acid. This is especially valuable for elderly persons who are often deficient in these minerals.

For administration to human patients appropriate dosages can routinely be determined by those skilled in this art by extension of the results obtained in animals at various dosages. The preferred dosage for humans falls within the range of 0.1 to 1000 mg, especially 0.1-200 mg $IP_3$/day/kg body weight.

In animal experiments, no toxic effects were seen after administration of very high doses of $IP_3$, 160 mg/kg body weight by intraperitoneal injection to mice.

The medicament usually contains 0.01-1.5 g, such as 0.05-1.3 or preferably 0.1-1 g of $IP_3$ per unit dosage.

In one preferred embodiment of the invention the isomer of $IP_3$ is D-myo-inositol-1.2.6-trisphosphate.

One type of function of the medicament is to reverse, prevent or alleviate damage to membranes of different cell types, but especially cell membranes of platelets, erythrocytes and endothelial cells. The use of the medicament results in an improved stabilization, a decreased susceptibility to deformation and an improved function of the cell types. Furthermore, parameters such as aggregability and adhesion to e.g. vessel walls are decreased.

The use of the medicament reduces lipid peroxidation of for example cell membranes.

Furthermore a normalization of the inositol metabolism is obtained when the medicament is used. Other results of the use of the medicament are regulation of membrane fluidity, the incorporation of components such as cholesterol and the production, incorporation and balance between different phospholipids.

Another result of the use of the medicament is the regulation of the electrolyte balance of the cell. As examples, regulation of intracellular and extracellular levels of calcium, potassium, chloride, phosphate etc. can be mentioned.

The cell surface activity and responsiveness to external stimuli via receptor activation /deactivation due to for example phosphorylation are other parameters effected by the use of the medicament.

The invention will be further explained with the following examples. Examples 1-4 show the production of $IP_3$ whereas examples 5 and 6 illustrate the preparation of solutions and tablets of $IP_3$. Examples 7-10 describe the effect of $IP_3$ on disorders related to transplantation.

Example 1

Hydrolysis of sodium phytate with baker's yeast and fractionation of a mixture of inositol phosphates.

A 1.4 kg quantity of sodium phytate (from corn, Sigma Chemical Co) was dissolved in 1 200 l sodium acetate buffer pH 4.6. 100 kg of baker's yeast from Jästbolaget, Sweden (dry substance: 28 %, nitrogen content: 2 %, phosphorus content: 0.4 %) was added with stirring and incubation was continued at 45°C. The dephosphorylation was followed by determining the inorganic phosphorus released. After 7 hours when 50 % inorganic phosphorus was liberated the hydrolysis was stopped by adding ammonia to pH 12. The suspension was centrifuged and the supernatant was collected.

800 l of the supernatant was passed through an ion-exchange column (Dowex 1, chloride form, 100 cm x 150 cm) and eluted with a linear gradient of hydrochloric acid (0-0.7 N HCL).

## Example 2

Structural determination of isomers of inositol triphosphate.

The fraction obtained in example 1 with a phosphorus/ inositol ratio of three to one was neutralized and precipitated as a calcium salt. 100 mg of the precipitate was analyzed with H-NMR. Data show that the peak consists of myo-inositol-1.2.6-trisphosphate.

## Example 3

A 0.5 gram quantity of D-chiro-inositol was dissolved in 1 ml phosphoric acid at 60°C. 20 g polyphosphoric acid was added and the mixture was heated to 150°C under vacuum for 6 hours. The mixture was diluted with water to a volume of 200 ml and passed through an ion-exchange column (Dowex 1, chloride form, 25 mm x 250 mm) and eluted with a linear gradient of hydrochloric acid (0-2.0 N HCl).

The content of the peak with the ratio of phosphorus to inositol of six to one was precipitated by addition of calciumhydroxide. The precipitate was filtered, washed and mixed with 10 ml of a cation-exchange resin to give the acid form of the inositolhexaphosphate. After neutralization with sodium hydroxide and freeze-drying the sodium salt of D-chiro-inositolhexaphosphate was obtained.

## Example 4

A 0.8 gram quantity of the sodium salt of D-chiro-inositolhexaphosphate produced according to Example 3 was dissolved in 300 ml sodium acetate buffer, pH 5.2. 1.3 gram wheat phytase (EC 3.1.3.26, 0.015 U/mg from Sigma Chemical Co.) was added and the mixture was incubated at 38°C.

After the liberation of 50 % inorganic phosphorus the hydrolysis was stopped by adding ammonia to pH 12.

The mixture containing D-chiro-inositolphosphates was passed through an ion-exchange column (Dowex 1 chloride form, 25 mm x 250 mm) and eluted with a linear gradient of hydrochloric acid (0-0.7 N HCL).

The peak with the ratio of phosphorus to inositol of three to one was neutralized with 1.0 M sodium hydroxide and freeze-dried.

Structural determination with NMR and IR showed the product to be D-chiro-inositoltriphosphate.

## Example 5

Solution of sodium salt of D-myo-inositol-1.2.6-trisphosphate for injection.

0.5 g of the sodium salt of $IP_3$ and 0.77 g NaCl were dissolved in 98.73 ml of water for injection to form a solution suitable for injection into a person or an animal.

## Example 6

Tablets of calcium salt of D-myo-inositol-1.2.6-trisphosphate.

Tablets of the calcium salt of D-myo-inositol-1.2.6-trisphosphate were produced in the following way. 50 g calcium salt of D-myo-inositol-1.2.6-triphosphate, 132 g lactose and 6 g acacia were mixed. Purified water was then added to the mixture, whereupon the mixing was continued until a suitable consistency was obtained. The mixture was sieved and dried. Then the mixture was blended with 10 g talcum and 2 g magnesium stearate. The mixture was compressed into tablets each weighing 200 mg.

## Example 7

Transplantation of an organ from one animal to another includes first a reduction of blood flow followed by reflow of blood in the new animal. During this process the organ is easily damaged and as a measurement the so called Schiff base is determined.

In one group of six kidneys from rabbits the blood flow was first reduced for 120 minutes followed by reflow for 60 minutes. The other group of six kidneys was processed in the same way but two doses of 50 ppm D-myo-inositol-1.2.6-trisphosphate ($IP_3$) were administered 5 minutes before flow reduction and 5 minutes before reflow. Schiff base was determined in cortex and medulla of the kidneys (% of control):

|  | Schiff base | |
| --- | --- | --- |
| Treatment | Cortex | Medulla |
| Control, group 1 | 100 | 100 |
| IP$_3$, group 2 | 20 | 45 |

Thus, IP$_3$-treated kidneys are strongly protected against damage during transplantation.

Example 8

Kidneys were harvested from rabbits, stored for 48 hours and then operated back into the same animal. Kidneys from 10 rabbits, group 1, were flushed at a constant pressure (100 mg Hg) via the renal artery with cold (4°C) hypertonic citrate, placed in a beaker containing the same solution and stored at 0°C, surrounded by ice in a polystyrene container for a period of 48 hours.

Kidneys from another 10 rabbits, group 2, were treated under the same conditions except for the addition of 1 mM D-myo-inositol-1.2.6-trisphosphate (IP$_3$) to the hypertonic citrate solution. After the storage period all kidneys were transplanted back to the donor animals and after 60 min of reflow of blood the damage of the organs were determined. The amount of Schiff base formed, which reflects the damage of the organs, was determined in cortex and medulla of the kidneys (% of control):

|  | Schiff base (%) | |
| --- | --- | --- |
| Treatment | Cortex | Medulla |
| Control, group 1 | 100 | 100 |
| IP$_3$, group 2 | 15 | 24 |

The addition of IP$_3$ to the storage solution markedly reduces damages to different parts of kidneys at storage.

Example 9

Kidneys were allografted between two incompatible breeds of rabbits (Sandy Lop to New Zealand White).

One pair of rabbits served as a control, while another pair received D-myo-inositol-1.2.6-trisphosphate (IP$_3$).

To the donor animals were administered either 0.9% NaCl solution intravenously (control) or IP$_3$ in 0.9% NaCl solution at a dose of 50 mg/kg. After harvesting the kidneys from the donors the organs were flushed at a constant pressure (100 mg Hg) via the renal artery with cold (4°C) hypertonic citrate, placed in a beaker of 100 ml containing the same solution and stored at 0°C surrounded by ice in a polystyrene container for a period of 24 hours.

The recipients were given either 0.9% NaCl solution intravenously (control) or IP$_3$ in 0.9% NaCl solution at a dose of 50 mg/kg for 5 days after the kidney transplantation. The kidney in the control group ceased to pass urine by day 6 and had to be killed by day 9.

The animal receiving IP$_3$ was killed on day 29 but still had good renal function at that time. The results show that IP$_3$ had significant immunosuppressant properties and improved very dramatically the function of allografted kidneys.

Example 10

Skin were allografted between two incompatible breeds of rabbits (Sandy Lop to New Zealand White).

Seven pairs of rabbits served as control while another seven pairs of rabbits received D-myo-inositol-1.2.6-trisphosphate (IP$_3$).

The donor animals were treated with either 0.9% NaCl solution intravenously (control) or 50 mg/kg IP$_3$ in

0.9% NaCl solution 10 minutes before harvesting the skin fragments. Recipients were given either 0.9% NaCl solution intravenously (control) or 50 mg/kg IP$_3$ before transplantation and then for 5 days after the operation. The skin grafts in the control animals were totally rejected after a period of 10 days while the skin grafts in the IP$_3$-treated animals showed no signs of rejection when the experiment was terminated after 17 days.

The results show that IP$_3$-treatment significantly improves the function and counteracts rejection after skin grafting.

## Claims

1. The use of at least one isomer of inositoltrisphosphate (IP$_3$) for the preparing of a medicament for preventing, alleviating or combatting disorders related to transplantations in mammals including man.

2. The use according to claim 1, wherein the medicament reduces the rejection of the transplanted organ.

3. The use according to claim 1, wherein the transplantations relate to transplantation of skin.

4. The use according to any one of claims 1-3, wherein said inositoltrisphosphate is in salt form.

5. The use according to claim 4, wherein said inositoltrisphosphate salt is a salt of sodium, potassium, calcium or zinc.

6. The use according to any one of claims 1-5, wherein the medicament is in tablet or granulated form.

7. The use according to any one of claims 1-5, wherein the medicament is in the form of a solution.

8. The use according to any one of claims 1-7, wherein said inositoltrisphosphate is D-myo-inositol-1.2.6-trisphosphate.

## Patentansprüche

1. Verwendung von zumindest einem Isomer von Inositoltrisphosphat (Ip$_3$) für die Herstellung eines Medikamentes für die Verhinderung, Milderung oder das Bekämpfen von Krankheiten, die mit Transplantationen bei Säugern, einschließlich Menschen, in Beziehung stehen.

2. Verwendung nach Anspruch 1, dadurch gekennzeichnet, daß das Medikament die Abstoßung des transplantierten Organs vermindert.

3. Verwendung nach Anspruch 1, dadurch gekennzeichnet, daß die Transplantationen Hauttransplantationen betreffen.

4. Verwendung nach einem der Ansprüche 1 bis 3, dadurch gekennzeichnet, daß Innositoltrisphosphat in einer Salzform vorliegt.

5. Verwendung nach Anspruch 4, dadurch gekennzeichnet, daß das Inositoltrisphosphatsalz ein Salz von Natrium, Kalium, Kalzium oder Zink ist.

6. Verwendung nach einem der Ansprüche 1 bis 5, dadurch gekennzeichnet, daß das Medikament in Tabletten oder granulierter Form vorliegt.

7. Verwendung nach einem der Ansprüche 1 bis 5, dadurch gekennzeichnet, daß das Medikament in der Form einer Lösung vorliegt.

8. Verwendung nach einem der Ansprüche 1 bis 7, dadurch gekennzeichnet, daß das Inositoltriphosphat D-myo-Inositol-1,2,6-triphosphat ist.

## Revendications

1. Utilisation d'au moins un isomère du trisphosphate d'inositol (IP3) pour la préparation d'un médicament pour prévenir, soulager ou combattre les troubles liés aux transplantations chez les mammifères, y compris l'homme.

2. Utilisation selon la revendication 1, dans laquelle le médicament réduit le rejet de l'organe transplanté.

3. Utilisation selon la revendication 1, dans laquelle les transplantations sont des transplantations de peau.

4. Utilisation selon l'une quelconque des revendications 1 à 3, dans laquelle ce trisphosphate d'inositol est sous forme de sel.

5. Utilisation selon la revendication 4, dans laquelle ce sel de trisphosphate d'inositol est un sel de sodium, de potassium, de calcium ou de zinc.

6. Utilisation selon l'une quelconque des revendications 1 à 5, dans laquelle le médicament est sous forme de comprimé ou de granulé.

7. Utilisation selon l'une quelconque des revendications 1 à 5, dans laquelle le médicament est sois la

**EP 0 359 259 B1**

forme d'une solution.

8. Utilisation selon l'une quelconque des revendications 1 à 7, dans laquelle ce trisphosphate d'inositol est le 1,2,6-trisphosphate de D-myo-inositol.

**EP 0 359 259 B1**

forme d'une solution.

8. Utilisation selon l'une quelconque des revendications 1 à 7, dans laquelle ce trisphosphate d'inositol est le 1,2,6-trisphosphate de D-myo-inositol.

7